# EUROPEAN PATENT SPECIFICATION

(11) **EP 3 952 829 B1**
(45) Date of publication and mention of the grant of the patent: **02.11.2022**
(21) Application number: 20703158.4
(22) Date of filing: 23.01.2020
(51) Int. Cl.: A61K 8/34, A61Q 11/00

(54) **PROCESS FOR PRODUCING AN ORAL CARE COMPOSITION COMPRISING GINGERDIOL**
VERFAHREN ZUR HERSTELLUNG EINER MUNDPFLEGEZUSAMMENSETZUNG ENTHALTEND GINGERDIOL
PROCÉDÉ DE PRÉPARATION D'UNE COMPOSITION DE SOINS BUCCO-DENTAIRES COMPRENANT DU GINGERDIOL

(30) Priority: 08.04.2019 EP 19167838
(43) Date of publication of application: 16.02.2022
(73) Proprietor: UNILEVER GLOBAL IP LIMITED, Wirral Merseyside CH62 4ZD (GB); Unilever IP Holdings B.V., 3013 AL Rotterdam (NL)
(72) Inventor: GOLDING, Stephen, Wirral Merseyside CH63 3JW (GB); LITTLEWOOD, David, Thomas, Wirral Merseyside CH63 3JW (GB); MARRIOTT, Robert, Edward, Wirral Merseyside CH63 3JW (GB); SKINNER, Richard, Wirral Merseyside CH63 3JW (GB)
(74) Representative: Tansley, Sally Elizabeth
(86) International application number: PCT/EP2020/051635
(87) International publication number: WO 2020/207633

(56) References cited:
- WO-A1-2018/041544
- US-A- 5 037 633
- US-A1- 2018 021 231

## Description

The present invention relates to a process for preparing an oral care composition, more particularly a composition for application to a topical surface or the oral cavity.

### Background of the Invention

Many products that we consume have a negative impact on our teeth and mouth. Acidic drinks and sweets, for example, can result in tooth erosion by attacking enamel which is the outer coating that protects the teeth. Foods which are sticky can accumulate in the interstices between teeth and in the region between the gums and the teeth. In the absence of good oral hygiene, these can harden over time with the help of oral bacteria to form a film known as plaque. Gingivitis is an inflammatory process of the gums caused by accumulation of plaque and/or bacteria. During gingivitis, the bacteria residing in the dental plaque biofilms and its corresponding components interact with gingival tissues. Gingivitis is a mild phase of periodontal disease and defined as reversible inflammation. It is believed that good habit of oral hygiene e.g. brushing and using a mouthrinse product with therapeutic anti-microbial and anti-inflammatory efficacy can be an effective way for individuals to reduce gingivitis. Chronic gingivitis results in mild bleeding from the gums during tooth brushing. Gingivitis may progress to a more severe state (chronic periodontitis) when the inflammatory process extends to the periodontal ligament and alveolar bone and/or exert a significant systemic impact on health. Chronic periodontitis is asymptomatic until teeth shift, loosen, or are lost.

KR100835899 (Korean Res. Inst of Bioscience, 2008) discloses an extract of Zingiber officinale Roscoe or gingerol isolated therefrom is provided to show antibacterial capability and sterilising capability thereby being usefully used for preventing or treating periodontal diseases. WO2018/041544 discloses a combination of gingerdiol and vitamin B3 as an active for reducing inflammation which may be used in oral, scalp/hair and skin care products.

However, to produce an oral care composition in which the gingerdiol is evenly distributed throughout the product is difficult. The present invention discloses a process of manufacture in which the gingerdiol is evenly distributed throughout the product.

### Description of the Invention

The present invention relates to a process for producing an oral care composition comprising gingerdiol the process comprising the following steps:
i) adding gingerdiol to a polyol or a polyol mixture comprising less than 5 wt% of the polyol mixture of water, to form a gingerdiol pre-mix in the form of a suspension or solution
ii) adding the resulting pre-mix to a liquid base composition.

### Detailed Description of the Invention

Compositions prepared according to invention comprise gingerdiol.

Gingerdiol is also know by the chemical name 1-(4-hydroxy-3-methoxyphenyl)decane3,5-diol.

Gingerdiol for use in the composition of the invention has to be synthesized in a chemical reaction. A suitable chemical synthesis process comprises the steps of taking gingerol in a suitable solvent and a reducing agent at a temperature of 0 to 10 °C; warming the reaction mixture to 15 to 25 °C to form the gingerdiol; and separating the gingerdiol from the mixture using solvent extraction.

The gingerdiol of the invention has the following chemical structure as shown below:

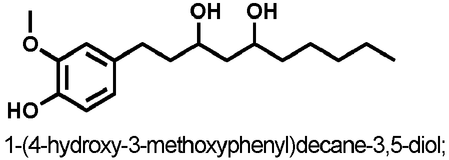

The various possible chemicals and process conditions which may be used in the preparation of gingerdiol are described in WO2018/041544.

It is preferable if the gingerdiol is present in the total composition at a level from 0.01 wt% to 5wt%, more preferably from 0.05 wt% to 2wt%, most preferably at a level from 0.1wt% to 1wt%.

Preferably the weight ratio of gingerdiol to polyol in the pre-mix is from 2:1 to 1:15, more preferably from 1:1 to 1:10, most preferably from 1:2 to 1:6.

Preferably the gingerdiol is added to a polyol or polyol mixture to form a pre-mix at a temperature of 40°C to 90°C, more preferably 50°C to 70°C, these temperatures are particularly preferred if the polyol is glycerol.

The base composition preferably comprises water and/or sorbitol or when the composition in non aqueous glycerol.

A preferred process comprising the following steps:
i) adding gingerdiol to a polyol or a polyol mixture comprising less than 5 wt% of the polyol mixture of water, to form a gingerdiol pre-mix in the form of a suspension or solution;
ii) forming a liquid base composition comprising a polyol and particulate;
iii) adding the gingerdiol pre-mix to the liquid base composition;
iv) adding further particulate to the resulting composition.

The base composition preferably comprises particulates material, preferably the particulate material comprises silica. It is preferable if the level of silica in the pre-treatment composition i) is from 20 to 80 wt% of the total level of the silica in the composition, more preferably from 30 to 70 wt, most preferably from 40 to 60 wt%.

If present it is preferable if surfactant is added with the further particulate in step iv).

In a preferred embodiment the polyol of the gingerdiol pre-mix comprises glycerol. It is preferred if the poylol mixture which is used to dissolve the gingerdiol to form the pre-mix comprises at least 95 wt% of the total polyol mixture of a single polyol, more preferably 99 wt%.

The composition prepared according to the process of the invention is an oral care composition, preferably it is used to clean the surfaces of the oral cavity and is known as an oral care composition.

Accordingly, preferred product forms for compositions prepared according to the process of the invention are those which are suitable for brushing and/or rinsing the surfaces of the oral cavity.

The composition prepared according to the process of the invention is most preferably in the form of a dentifrice. The term "dentifrice' denotes an oral composition which is used to clean the surfaces of the oral cavity. Such a composition is not intentionally swallowed for purposes of systemic administration of therapeutic agents, but is applied to the oral cavity, used to treat the oral cavity and then expectorated. Typically, such a composition is used in conjunction with a cleaning implement such as a toothbrush, usually by applying it to the bristles of the toothbrush and then brushing the accessible surfaces of the oral cavity.

Preferably the dentifrice/toothpaste is in the form of an extrudable semi-solid such as a cream, paste or gel (or mixture thereof).

Preferably the liquid base comprises water, it is also preferable if the liquid base comprises sorbitol.

In a dentifrice the combination of sorbitol and water is preferred.

In some embodiments the level of water is from 15 to 50 wt% of the total composition, in other embodiments the level of water is kept to a minimum such compositions are known as nonaqueous. In non aqueous compositions the level of water is below 0.5 wt% of the total composition.

A composition prepared according to the process of the invention (such as a dentifrice/toothpaste) will generally contain further ingredients to enhance performance and/or consumer acceptability, in addition to the ingredients specified above.

Compositions prepared according to the process of the invention, particularly toothpastes, preferably comprise particulate abrasive materials such as silicas, aluminas, calcium carbonates, dicalciumphosphates, calcium pyrophosphates, hydroxyapatites, trimetaphosphates, insoluble hexametaphosphates and so on, including agglomerated particulate abrasive materials, usually in amounts between 3 and 60% by weight of the oral care composition.

Preferably the composition, particularly a toothpaste, comprises a silica based abrasive. The preferred abrasive silicas used in the present invention is a silica with a low refractive index. It may be used as the sole abrasive silica, or in conjunction with a low level of other abrasive silicas, e.g. those according to EP 236070. The low refractive index silicas, used as abrasives in the present invention are preferably silicas with an apparent refractive index (R.I.) in the range of 1.41 -1.47, preferably 1.435 - 1.445, preferably having a weight mean particle size of between 5 and 15 mm, a BET (nitrogen) surface area of between 10 and 100 m²/g and an oil absorption of about 70 - 150 cm³/100 g, but abrasive silicas with a lower apparent refractive index may also be used. Typical examples of suitable low refractive index abrasive silicas (e.g. having an R.I. of between 1.435 and 1.445) are Tixosil 63 and 73 ex Rhone Poulenc; Sident 10 ex Degussa; Zeodent 113 ex Zeofinn; Zeodent 124 ex Huber, Sorbosil AC 77 ex Crosfield Chemicals (having an R.I. of approximately 1.440). The amount of these silicas in the composition generally ranges from 5-60% by weight, usually 5-20% by weight.

The composition, particularly if a toothpaste preferably comprises an inorganic or a natural or synthetic thickener or gelling agent in proportions of about 0.10 to about 15% by weight depending on the material chosen. These proportions of thickeners in the dentifrice compositions prepared according to the process of the present invention form an extrudable, shape-retaining product which can be squeezed from a tube onto a toothbrush and will not fall between the bristles of the brush but rather, will substantially maintain its shape thereon. Suitable thickeners or gelling agents useful in the practice of the present invention include inorganic thickening silicas such as amorphous silicas available from Huber Corporation under the trade designation Zeodent 165, Irish moss, iota-carrageenan, gum tragacanth, and polyvinylpyrrolidone.

Compositions prepared according to the process of the invention preferably comprise a polymeric deposition aid. Preferably the composition comprises acid anhydride polymers, particularly preferred are co-polymers of maleic anhydride with methyl vinylether, in which the anhydride moiety may be in a partially or fully hydrolysed or alcoholysed form. Preferred copolymers include Gantrez(R) polymers such as:
Gantrez S-95: molecular weight 216,000; free acid;
Gantrez S-96: molecular weight 700,000; free acid;
Gantrez S-97: molecular weight 1,500,000; free acid; and
Gantrez MS-955: molecular weight 1,060,000; calcium/sodium salt.

Particularly preferred co-polymers of maleic acid and methyl vinylether have a molecular weight of 1,000,000 or greater and an especially preferred material is Gantrez S-97.

Compositions prepared according to the process of the invention may comprise a tooth whitening agent. The whitening agent preferably comprises a green and/or a blue pigment. In the context of the present invention a pigment is generally understood to be a shade/material which is insoluble in the relevant medium, at the relevant temperature. This is in contrast to dyes which are soluble. In the context of this invention, the "relevant medium" is human saliva, the liquid medium in which the composition is used, at the temperature of the oral cavity during brushing of the teeth, i.e. up to 37 Degrees C. As a reasonable approximation, the relevant medium may be considered to be water and the relevant temperature to be 25 Degrees C.

Preferably the blue pigment is Pigment Blue 15, more preferably Pigment Blue 15:1, 15:2, 15:3, 15:4, 15:5 or 15:6, most preferably 15:1. A preferred pigment is blue pigment is Phthalocyanine Blue Pigment, CI No. 74160, blue covarine.

The preferred Green pigment is Phthalocyanine Green, preferably Phthalocyanine Green CI-74260.

Preferably the total level of pigment in the composition is from 0.01 wt% to 3 wt, more preferably from 0.02 to 2 wt%.

If the composition is a toothpaste it may be a dual phase paste, with the whitening pigments present in one phase.

Compositions prepared according to the process of the invention may comprise water-soluble or sparingly water-soluble sources of zinc ions such as zinc chloride, zinc acetate, zinc gluconate, zinc sulphate, zinc fluoride, zinc citrate, zinc lactate, zinc oxide, zinc monoglycerolate, zinc tartrate, zinc pyrophosphate and zinc maleate;

Compositions prepared according to the process of the invention may comprise oral care enzyme systems such as hydrogen peroxide producing enzyme systems (e.g. the oxidoreductase enzyme glucose oxidase), amyloglucosidase, dextranase and/or mutanase, (optionally in the presence of zinc ion providing compounds and/or 8- hydroxyquinoline derivatives), lactoperoxidase, lactoferrin, lysozyme and mixtures thereof;

Compositions prepared according to the process of the invention may comprise fluoride sources such as sodium fluoride, stannous fluoride, stannous pyrophosphate, sodium monofluorophosphate, zinc ammonium fluoride, tin ammonium fluoride, calcium fluoride, cobalt ammonium fluoride and mixtures thereof;

In one embodiment a preferred class of oral care active for inclusion in the compositions prepared according to the process of the invention includes agents for the remineralisation of teeth. The term "remineralisation" in the context of the present invention means the *in situ* generation of hydroxyapatite on teeth.

A specific example of a suitable agent for the remineralisation of teeth is a mixture of a calcium source and a phosphate source which, when delivered to the teeth results in the *in situ* generation of hydroxyapatite on teeth.

Illustrative examples of the types of calcium source that may be used in this context (hereinafter termed "remineralising calcium sources") include, for example, calcium phosphate, calcium gluconate, calcium oxide, calcium lactate, calcium glycerophosphate, calcium carbonate, calcium hydroxide, calcium sulphate, calcium carboxymethyl cellulose, calcium alginate, calcium salts of citric acid, calcium silicate and mixtures thereof. Preferably the remineralising calcium source is calcium silicate.

The amount of remineralising calcium source(s) (e.g. calcium silicate) in the composition prepared according to the process of the invention typically ranges from 1 to 30%, preferably from 5 to 20% by total weight remineralising calcium source based on the total weight of the oral care composition.

Illustrative examples of the types of phosphate source that may be used in this context (hereinafter termed "remineralising phosphate sources") include, for example, monosodium dihydrogen phosphate, disodium hydrogen phosphate, sodium pyrophosphate, tetrasodium pyrophosphate, sodium tripolyphosphate, sodium hexametaphosphate, potassium dihydrogenphosphate, trisodium phosphate, tripotassium phosphate and mixtures thereof. Preferably the remineralising phosphate source is a mixture of trisodium phosphate and sodium dihydrogen phosphate.

The amount of remineralising phosphate source(s) (e.g. trisodium phosphate and sodium dihydrogen phosphate) in the composition of this invention typically ranges from 2 to 15%, preferably from 4 to 10% by total weight remineralising phosphate source based on the total weight of the oral care composition.

Mixtures of any of the above described materials may also be used.

The composition prepared according to the process of the invention will comprise further ingredients which are common in the art, such as:
antimicrobial agents, e.g. chlorhexidine, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds, such as 2,2' methylenebis-(4-chloro-6-bromophenol);
anti-inflammatory agents such as ibuprofen, flurbiprofen, aspirin, indomethacin etc.;
anti-caries agents such as sodium- and stannous fluoride, aminefluorides, sodium monofluorophosphate, sodium trimeta phosphate and casein;
plaque buffers such as urea, calcium lactate, calcium glycerophosphate and strontium polyacrylates;
vitamins such as Vitamins A, C and E;
plant extracts;
plant-derivable antioxidants such as flavonoid, catechin, polyphenol, and tannin compounds and mixtures thereof;
desensitising agents, e.g. potassium citrate, potassium chloride, potassium tartrate, potassium bicarbonate, potassium oxalate, potassium nitrate and strontium salts;
anti-calculus agents, e.g. alkali-metal pyrophosphates, hypophosphite-containing polymers, organic phosphonates and phosphocitrates etc.;
biomolecules, e.g. bacteriocins, antibodies, enzymes, etc.;
flavours, e.g. peppermint and spearmint oils;
proteinaceous materials such as collagen;
preservatives;
opacifying agents;
amino acids such as arginine
colouring agents;
pH-adjusting agents;
sweetening agents;
pharmaceutically acceptable carriers, e.g. starch, sucrose, water or water/alcohol systems etc.; surfactants, such as anionic, nonionic, cationic and zwitterionic or amphoteric surfactants; Humectants such as glycerol, sorbitol, propyleneglycol, xylitol, lactitol etc.;
binders and thickeners such as sodium carboxymethyl-cellulose, hydroxyethyl cellulose (Natrosol^{®}), xanthan gum, gum arabic etc. as well as synthetic polymers such as polyacrylates and carboxyvinyl polymers such as Carbopol^{®};
polymeric compounds which can enhance the delivery of active ingredients such as antimicrobial agents can also be included;
buffers and salts to buffer the pH and ionic strength of the oral care composition; and other optional ingredients that may be included are e.g. bleaching agents such as peroxy compounds e.g. potassium peroxydiphosphate, effervescing systems such as sodium bicarbonate/citric acid systems and colour change systems.

The invention will now be illustrated by the following non-limiting Examples.

### Examples

A base composition was prepared by combining water + sorbitol followed by addition of TiO₂, saccharin and PEG 1500 and sodium fluoride to the mixture. This was followed by addition of silica and sodium carboxymethylcellulose.

Gingerdiol was added to a polyol listed in table 1 in a separate vessel. The mixture was heated to 65 °C and the mixture stirred. The resulting gingerdiol pre-mix was added to the base composition.

Finally, silica, surfactant and other minors were added.

The formulation of the Examples is as in Table 1:

**Table 1**

| **Chemical Name** | **Percentage in Formulation** | | | |
|---|---|---|---|---|
| | **Example 1** | **Example 2** | **Example 3** | **Example A** |
| Titanium Dioxide (Anatase) | 0.25 | 0.25 | 0.25 | 0.25 |
| Glycerin* | 1.11 | 0 | 0 | 0 |
| Glycerin** | | | | 1.11 |
| Propylene glycol* | 0 | 1.11 | 0 | 0 |
| 70%Sorbitol/30%water* | 0 | 0 | 1.11 | 0 |
| Flavour | 1.2 | 1.2 | 1.2 | 1.2 |
| Water | 31.2 | 31.2 | 31.2 | 31.2 |
| Synthetic Amorphous Silica | 16 | 16 | 16 | 16 |
| Polyethylene Glycol | 2 | 2 | 2 | 2 |
| Sulphuric acid, mono C12-18-alkyl esters, sodium salts | 1.7 | 1.7 | 1.7 | 1.7 |
| Sodium Fluoride | 0.32 | 0.32 | 0.32 | 0.32 |
| 1-(4-hydroxy-3-methoxyphenyl)decane-3,5-diol; Gingerdiol | 0.3 | 0.3 | 0.3 | 0.3 |
| Sodium Carboxymethylcellulose | 0.7 | 0.7 | 0.7 | 0.7 |
| Sorbitol and minors | To 100 | To 100 | To 100 | To 100 |

| | | | | |
|---|---|---|---|---|
| * added as pre-mix with gingerdiol. **in formulation | | | | |

The ease of formulation is stated in Table 2.

**Table 2**

| **Example** | **Results** |
|---|---|
| 1 | Even dispersion of gingerdiol in toothpaste |
| 3 | Dispersion of gingerdiol not so even in toothpaste |
| 2 | Even dispersion of gingerdiol in toothpaste formulation |
| A | Difficult to formulate did not disperse |

The results of Table 2 show that it is easier to formulate a composition with the Examples according to the invention than the comparative Example.

## Claims

1. A process for producing an oral care composition comprising gingerdiol the process comprising the following steps:
i) adding gingerdiol to a polyol or a polyol mixture comprising less than 5 wt% of the polyol mixture of water, to form a gingerdiol pre-mix in the form of a suspension or solution;
ii) adding the resulting suspension or solution to a liquid base composition.

2. A process according to claim 1 in which the polyol or polyol mixture comprises glycerol.

3. A process according to claim 1 or claim 2 in which the polyol or polyol mixture is at a temperature of 50°C to 90°C on addition or after the addition of the gingerdiol.

4. A process according to any preceding claim in which the poylol mixture comprises at least 95 wt% of the total polyol mixture of a single polyol.

5. A process according to any preceding claim in which the level of gingerdiol present in the total composition is from 0.01 wt% to 5wt%, preferably 0.05 wt% to 2wt% more preferably 0.1 wt% to 1 wt%.

6. A process according to any preceding claim in which the weight ratio of gingerdiol to polyol in the pre-mix is from 2:1 to 1:15, preferably from 1:1 to 1:10, more preferably from 1:2 to 1:6.

7. A process according to any preceding claim in which the liquid base composition comprises water and sorbitol.

8. A process according to claim 7 in which the level of water in the total composition is from 15 to 50 wt% of the total composition.

9. A process according to any preceding claim in which the composition comprises a particulate.

10. A process according to any preceding claim in which the liquid base composition comprises a particulate.

11. A process according to any one of claims 9 and 10 in which the particulate comprises silica.

12. A process as claimed in any one of claims 9 to 11 in which part of the particulate is added to the liquid base composition after the addition of the gingerdiol.

13. A process according to any preceding claim in which the oral care composition is a dentifrice.

## Patentansprüche

1. Verfahren zur Herstellung einer Mundpflegezusammensetzung, umfassend Gingerdiol, wobei das Verfahren die folgenden Schritte umfasst:
i) Zugabe von Gingerdiol zu einem Polyol oder einer Polyolmischung, umfassend weniger als 5 Gew.-% Wasser, bezogen auf die Polyolmischung, um eine Gingerdiolvormischung in Form einer Suspension oder einer Lösung zu bilden;
ii) Zugabe der erhaltenen Suspension oder Lösung zu einer flüssigen Basiszusammensetzung.

2. Verfahren nach Anspruch 1, bei dem das Polyol oder die Polyolmischung Glycerin umfasst.

3. Verfahren nach Anspruch 1 oder Anspruch 2, bei dem das Polyol oder die Polyolmischung sich bei einer Temperatur von 50°C bis 90°C bei der Zugabe oder nach der Zugabe des Gingerdiols befindet.

4. Verfahren nach einem vorhergehenden Anspruch, bei dem die Polyolmischung mindestens 95 Gew.-% der gesamten Polyolmischung eines einzelnen Polyols umfasst.

5. Verfahren nach einem vorhergehenden Anspruch, bei dem der in der gesamten Zusammensetzung vorliegende Gehalt an Gingerdiol von 0,01 Gew.-% bis 5 Gew.-%, vorzugsweise von 0,05 Gew.-% bis 2 Gew.-%, bevorzugter von 0,1 Gew.-% bis 1 Gew.-%, beträgt.

6. Verfahren nach einem vorhergehenden Anspruch, in welchem das Gewichtsverhältnis von Gingerdiol zu Polyol in der Vormischung von 2:1 bis 1:15, vorzugsweise von 1:1 bis 1:10, bevorzugter von 1:2 bis 1:6, beträgt.

7. Verfahren nach irgendeinem vorhergehenden Anspruch, bei dem die flüssige Basiszusammensetzung Wasser und Sorbit umfasst.

8. Verfahren nach Anspruch 7, in dem der Gehalt an Wasser in der gesamten Zusammensetzung 15 bis 50 Gew.-% der gesamten Zusammensetzung beträgt.

9. Verfahren nach einem vorhergehenden Anspruch, bei dem die Zusammensetzung ein teilchenförmiges Material umfasst.

10. Verfahren nach einem vorhergehenden Anspruch, in welchem die flüssige Basiszusammensetzung ein teilchenförmiges Material umfasst.

11. Verfahren nach einem der Ansprüche 9 und 10, in welchem das teilchenförmige Material Siliziumdioxid umfasst.

12. Verfahren, wie in irgendeinem der Ansprüche 9 bis 11 beansprucht, bei dem ein Teil des teilchenförmigen Materials nach der Zugabe des Gingerdiols zu der flüssigen Basiszusammensetzung gegeben wird.

13. Verfahren nach irgendeinem vorhergehenden Anspruch, in welchem die Mundpflegezusammensetzung ein Zahnputzmittel ist.

## Revendications

1. Procédé pour la production d'une composition pour le soin oral comprenant du gingerdiol, le procédé comprenant les étapes suivantes :
i) addition de gingerdiol à un polyol ou un mélange de polyols comprenant pour moins de 5 % en masse du mélange de polyol de l'eau, pour former un pré-mélange de gingerdiol dans la forme d'une suspension ou solution ;
ii) addition de la suspension ou solution résultante à une composition de base liquide.

2. Procédé selon la revendication 1, dans lequel le polyol ou mélange de polyols comprend du glycérol.

3. Procédé selon la revendication 1 ou revendication 2, dans lequel le polyol ou mélange de polyols est à une température de 50°C à 90°C lors de l'addition ou après l'addition du gingerdiol.

4. Procédé selon l'une quelconque des revendications précédentes, dans lequel le mélange de polyols comprend pour au moins 95 % en masse du mélange total de polyols d'un polyol unique.

5. Procédé selon l'une quelconque des revendications précédentes, dans lequel la teneur en gingerdiol présent dans la composition totale est de 0,01 % en masse à 5 % en masse, de préférence de 0,05 % en masse à 2 % en masse, encore mieux de 0,1 % en masse à 1 % en masse.

6. Procédé selon l'une quelconque des revendications précédentes, dans lequel le rapport en masse de gingerdiol à polyol dans le pré-mélange est de 2:1 à 1:15, de préférence de 1:1 à 1:10, encore mieux de 1:2 à 1:6.

7. Procédé selon l'une quelconque des revendications précédentes, dans lequel la composition de base liquide comprend de l'eau et du sorbitol.

8. Procédé selon la revendication 7, dans lequel la teneur en eau dans la composition totale est de 15 à 50 % en masse de la composition totale.

9. Procédé selon l'une quelconque des revendications précédentes, dans lequel la composition comprend une matière particulaire.

10. Procédé selon l'une quelconque des revendications précédentes, dans lequel la composition de base liquide comprend une matière particulaire.

11. Procédé selon l'une quelconque des revendications 9 et 10, dans lequel la matière particulaire comprend de la silice.

12. Procédé selon l'une quelconque des revendications 9 à 11, dans lequel une partie de la matière particulaire est ajoutée à la composition de base liquide après l'addition du gingerdiol.

13. Procédé selon l'une quelconque des revendications précédentes, dans lequel la composition pour le soin oral est un dentifrice.
